## Europäisches Patentamt
## European Patent Office
## Office européen des brevets

(19)

(11) Veröffentlichungsnummer: **0 063 340**
**B1**

(12) **EUROPÄISCHE PATENTSCHRIFT**

(45) Veröffentlichungstag der Patentschrift:
**21.11.84**

(21) Anmeldenummer: **82103078.0**

(22) Anmeldetag: **09.04.82**

(51) Int. Cl.³: **A 61 M 5/30**

(54) **Nadelloses Injektionsgerät.**

(30) Priorität: **16.04.81 DE 3115374**

(43) Veröffentlichungstag der Anmeldung:
**27.10.82 Patentblatt 82/43**

(45) Bekanntmachung des Hinweises auf die Patenterteilung:
**21.11.84 Patentblatt 84/47**

(84) Benannte Vertragsstaaten:
**CH DE FR GB LI**

(56) Entgegenhaltungen:
**US - A - 2 800 903**
**US - A - 3 057 349**

(73) Patentinhaber: **HOECHST AKTIENGESELLSCHAFT,
Postfach 80 03 20, D-6230 Frankfurt am Main 80 (DE)**

(72) Erfinder: **Dettbarn, Hans-Jürgen, Rehbocks Ecke 4,
D-3550 Marburg/Lahn (DE)**
Erfinder: **Zimmermann, Josef, Auf der Krautweide 11,
D-6231 Sulzbach (DE)**

## Beschreibung

Die Erfindung betrifft ein nadelloses Injektionsgerät mit einer Kolbenpumpe für das zu injizierende Medium, wobei die Kolbenpumpe mit einem Antriebsmotor verbunden ist, dessen Arbeitskolben, der sich auf einer Arbeitsfeder abstützt, verschiebbar in einer zylindrischen Bohrung des Motorgehäuses angeordnet ist, wobei in der zylindrischen Bohrung eine Druckkammer zur Aufnahme eines Druckmittels zum Spannen der Arbeitsfeder ausgebildet ist, wobei die Druckkammer Zu- und Abführeinrichtungen für das Druckmittel aufweist, wobei die Arbeitsfeder durch eine Klinke, die durch einen Riegel unterstützt wird, in gespannter Lage gehalten wird, und wobei sich der Riegel auf einem Bedienungselement abstützt.

Nach der deutschen Patentanschrift 1 080 267 ist ein nadelloses Injektionsgerät der genannten Art bekannt. Die durch Hydraulikflüssigkeit gespannten Arbeitsfedern werden durch eine Klinke, die durch einen Riegel unterstützt wird, in gespannter Lage gehalten. Der Riegel stützt sich auf einem Abzug ab. In der Versorgungseinrichtung und Zuführeinrichtung für die Hydraulikflüssigkeit ist ein Magnetventil angeordnet, dessen Stromkreis mit einem elektrischen Schalter verbunden ist, dessen Kontakte mit Hilfe eines Ventilnockens für das zu injizierende Medium betätigt werden. Nachteilig ist, daß die zusammengepreßten Federn durch die Hydraulikflüssigkeit in ihrer Lage gehalten werden müssen und daß unbeabsichtigtes Auslösen der Klinke und damit ein Abschuß der Injektion durch Betätigen des Abzugs zu jeder Zeit möglich ist.

Hier will die Erfindung Abhilfe schaffen. Die Erfindung, wie sie in den Ansprüchen gekennzeichnet ist, löst die Aufgabe dadurch, daß das Bedienungselement mit dem Riegel starr verbunden ist und der Riegel verschiebbar im Motorgehäuse angeordnet ist. Das Bedienungselement besteht aus einem federbelasteten Bedienungsknopf, der sich im Eingriff mit einer federbelasteten und konzentrisch zum Bedienungsknopf angeordneten Hülse befindet, wobei der Hülse ein teleskopartig gefederter Stößel und dem Bedienungsknopf ein starrer Stößel zugeordnet sind, die beiden Stößel in entsprechende Bohrungen im Bedienungselement geführt und durch Federn unterstützt werden, die Hülse, der Bedienungsknopf und das Motorgehäuse mit Ausnehmungen versehen sind, in die die Stößel wechselweise eingreifen, und der Hülse in axialer Richtung ein Stößel zum Betätigen der Zuführeinrichtung für das Druckmittel zugeordnet ist. Der teleskopartig gefederte Stößel kann Anschläge zum Begrenzen seiner Auslenkung aufweisen.

Die durch die Erfindung erreichten Vorteile sind im wesentlichen darin zu sehen, daß sichergestellt ist, daß die Injektion nur dann ausgelöst werden kann, wenn die Düse des Injektionsgerätes auf das zu injizierende Objekt gedrückt wird. Ein weiterer Vorteil liegt darin, daß während des Spannvorganges der Arbeitsfeder ein Auslösen der Injektion und umgekehrt während des Injizierens ein Spannen der Arbeitsfeder nicht möglich ist.

Im folgenden wird die Erfindung anhand von lediglich einen Ausführungsweg darstellenden Zeichnungen näher erläutert. Es zeigt

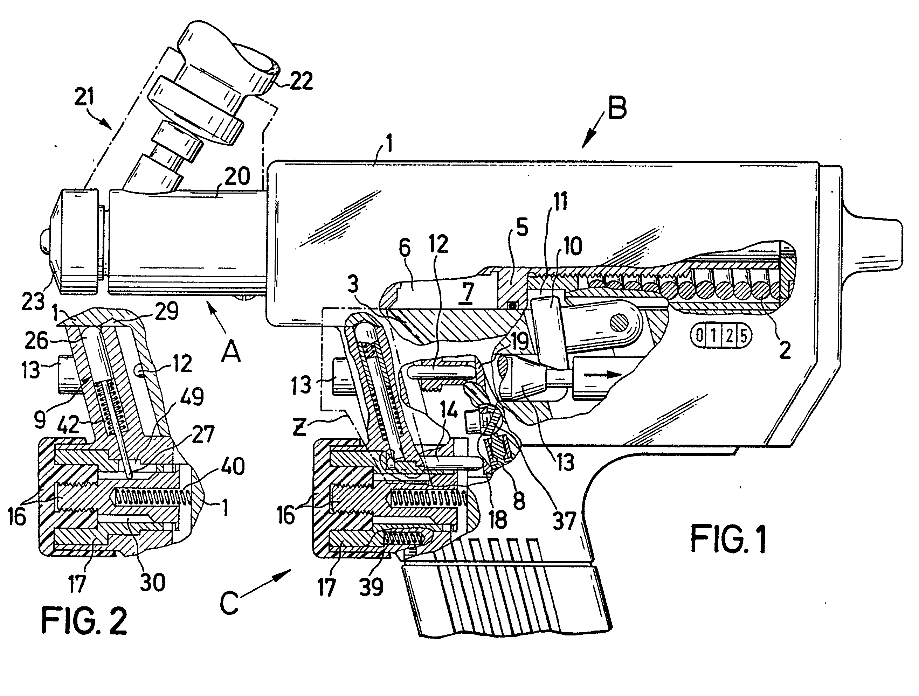

Fig. 1 eine Seitenansicht des Injektionsgerätes teilweise und in verschiedenen Ebene geschnitten in nahezu gespanntem Zustand; das Bedienungselement C ist noch ausgerückt, der Bedienungsknopf gedrückt, Teleskopstößel im Schnitt;

Fig. 2 das Bedienungselement noch ausgerückt, der Bedienungsknopf gedrückt, Starrstößel im Schnitt;

Fig. 3 eine Seitenansicht des Injektionsgerätes teilweise und in verschiedenen Ebenen geschnitten in gespanntem Zustand; das Bedienungselement (C) liegt am Motorgehäuse an; Bedienungsknopf ausgerückt;

Fig. 4 das Bedienungselement im Schnitt;

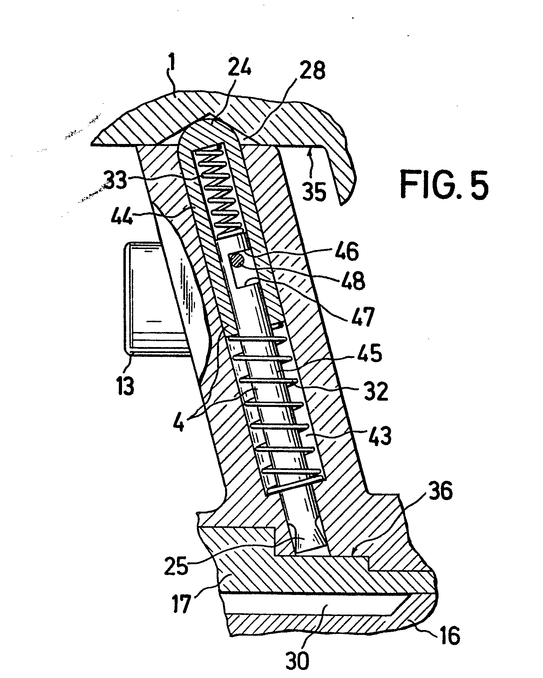

Fig. 5 die Einzelheit »Z« der Fig. 1.

Das nadellose Injektionsgerät besteht im wesentlichen aus einer Kolbenpumpe (A) für das zu injizierende Medium, die mit einem Antriebsmotor (B) verbunden ist. Das Gehäuse (20) der Kolbenpumpe (A) trägt eine Einrichtung (21) zur Aufnahme eines Behälters (22) für das zu injizierende Medium sowie die Düse (23). Der Arbeitskolben (5) des Antriebsmotors (B) ist in einer zylindrischen Bohrung (6) des Motorgehäuses (1) verschiebbar angeordnet. Die Arbeitsfeder (2) des Arbeitskolbens (5) wird mit einem Druckmittel, z. B. Hydraulikflüssigkeit oder Gas gespannt, das über Einlaßventil (8) und über Kanal (3) der Druckkammer (7) in der zylindrischen Bohrung (6) zugeführt wird. Das Bedienungselement (C) besteht im wesentlichen aus einem Bedienungsknopf (16), einer konzentrisch zum Bedienungsknopf angeordneten Hülse (17), einem starren Stößel (9) und einem teleskopartig gefederten Stößel (4). Die Stößel (9) und (4) sind in Bohrungen (42) und (43) des Bedienungselementes, z. B. einem Griff, federnd gelagert. Im Motorgehäuse (1) bzw. der Hülse (17) und dem Bedienungsknopf (16) sind Ausnehmungen (28) und (29) bzw. (30) und (31) angeordnet. Von den Ausnehmungen (29) bzw. (30) werden die Enden (26) bzw. (27) des Stößels (9) und von den Ausnehmungen (28) bzw. (31) die Enden (24) bzw. (25) des Stößels (4) aufgenommen. Die Stößel greifen wechselnd in die Ausnehmungen ein. Vor dem Spannen der Arbeitsfeder (2) fluchtet die Führungsbohrung (43) des federnden Stößels (4) mit der Ausnehmung (28) im Motorgehäuse, so daß der federnde Stößel (4) von der Feder (32) in die Ausnehmung (28) gedrückt wird. Das untere Ende (25) des Stößels (4) ist soweit angehoben, daß die Hülse (17) beim Drücken des Bedienungsknopfes (16) nach hinten gleiten kann. Der federnde Stößel (4) besteht aus einer Hülse (44), einem darin gleitenden Stempel (45) sowie einer Feder (33). Durch Anschläge (46) und (47) bzw. (48) an Stempel (45) bzw. Hülse (44) ist die Auslenkung des Stößels (4) begrenzt.

Die Führungsbohrung (42) des starren Stößels (9) fluchtet vor dem Spannen der Arbeitsfeder (2) nicht mit der Ausnehmung (29) im Motorgehäuse (1), so daß das obere Ende (26) des Stößels (9) an der planen Fläche (35) des Motorgehäuses anliegt und das untere Ende (27) des Stößels (9) durch einen Durchbruch (49) in der Hülse (17) in die Ausnehmung (30) des Bedienungsknopfes (16) ragt.

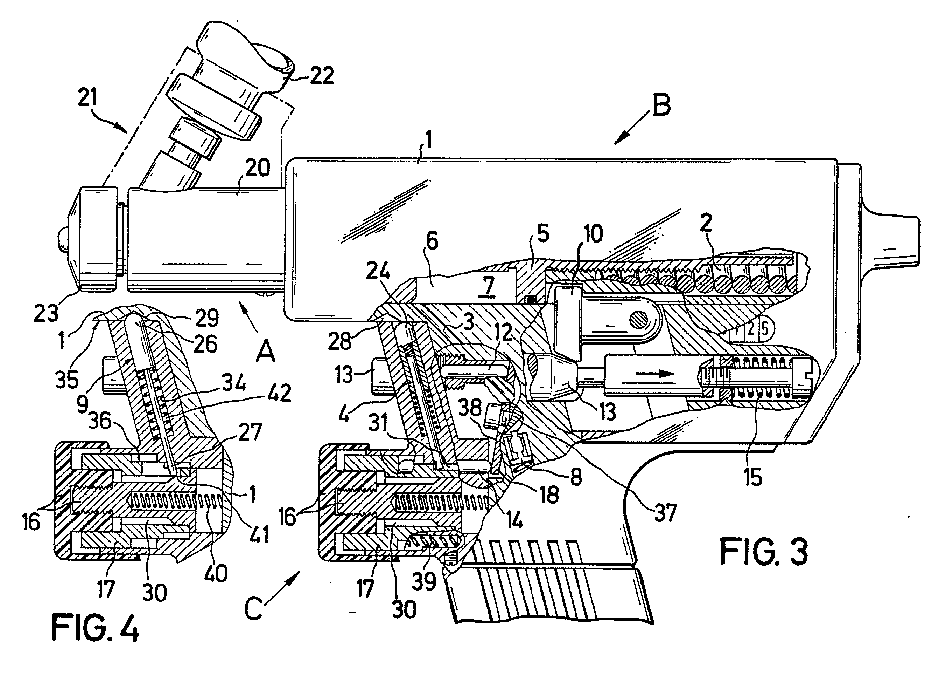

Beim Drücken des Bedienungsknopfes (16) wird auch die Hülse (17) zurückgeschoben. Die Hülse (17) drückt dabei auf den Stößel (14) und der wiederum auf das Ende (18) des Hebels (38); das Einlaßventil (8) wird geöffnet, die Arbeitsfeder (2) gespannt, die Ausnehmung (11) kommt gegenüber der Klinke (10) zu liegen, und der Riegel (13) wird durch die Feder (15) in Pfeilrichtung gezogen, ebenfalls das mit dem Riegel (13) starr verbundene Bedienungselement (C). Während des Zurückgleitens des Bedienungselementes (C) wird der Stößel (12) gedrückt, der am Ende (19) des Hebels (38) der Steuerwelle (37) angreift, und damit das Auslaßventil geöffnet, so daß das Druckmittel die Druckkammer (7) verlassen kann. Das obere Ende (24) des federnden Stößels (4) wird aus der Ausnehmung (28) geschoben, wobei das obere Ende (24) des Stößels (4) durch die Federn (32) und (33) gegen die Motorgehäusefläche (35) gedrückt wird, und das untere Ende (25) des Stößels (4) durch die Feder (33) auf die Fläche (36) der Hülse (17). Gleichzeitig wird der starre Stößel (9) in die Ausnehmung (29) im Motorgehäuse (1) durch die Feder (34) gedrückt, wobei das untere Ende (27) aus der Ausnehmung (30) rückt. Beim Loslassen des Bedienungsknopfes (16) wird durch Federn (39) die Hülse (17) und durch Feder (40) der Bedienungsknopf (16) in die Ausgangsstellung geschoben. Hierbei rutscht das untere Ende (25) des federnden Stößels (4) in die Ausnehmung (31) der Hülse (17) und verhindert ein erneutes Zurückschieben der Hülse (17) und damit ein erneutes Öffnen des Einlaßventils (8), solange die Impfpistole gespannt ist (Fig. 3). Beim Vorgleiten des Bedienungsknopfes (16) kommt die Schulter (41) dem unteren Ende (27) des Stößels (9) gegenüber zu liegen, so daß das Verschieben des Bedienungselementes (C) und somit die Auslösung der Injektion erst möglich wird, wenn der Bedienungsknopf (16) erneut gedrückt wird (Fig. 4).

## Patentansprüche

1. Nadelloses Injektionsgerät mit einer Kolbenpumpe (A) für das zu injizierende Medium, wobei die Kolbenpumpe (A) mit einem Antriebsmotor (B) verbunden ist, dessen Arbeitskolben (5), der sich auf einer Arbeitsfeder (2) abstützt, verschiebbar in einer zylindrischen Bohrung (6) des Motorgehäuses (1) angeordnet ist, wobei in der zylindrischen Bohrung (6) eine Druckkammer (7) zur Aufnahme eines Druckmittels zum Spannen der Arbeitsfeder (2) ausgebildet ist, wobei die Druckkammer (7) Zu- (3, 8) und Abführeinrichtungen für das Druckmittel aufweist, wobei die Arbeitsfeder (2) durch eine Klinke (10), die durch einen Riegel (13) unterstützt wird, in gespannter Lage gehalten wird, und wobei sich der Riegel (13) auf einem Bedienungselement (C) abstützt, dadurch gekennzeichnet, daß das Bedienungselement (C) mit dem Riegel (13) starr verbunden ist und der Riegel (13) verschiebbar im Motorgehäuse (1) angeordnet ist.

2. Nadelloses Injektionsgerät nach Anspruch 1, dadurch gekennzeichnet, daß das Bedienungselement (C) aus einem federbelasteten Bedienungsknopf (16) besteht, der sich im Eingriff mit einer federbelasteten und konzentrisch zum Bedienungsknopf (16) angeordneten Hülse (17) befindet, wobei der Hülse (17) ein teleskopartig gefederter Stößel (4) und dem Bedienungsknopf (16) ein starrer Stößel (9) zugeordnet sind, die beiden Stößel (4, 9) in entsprechenden Bohrungen (42, 43) im Bedienungselement (C) geführt und durch Federn (32, 33, 34) unterstützt werden, die Hülse (17), der Bedienungsknopf (16) und das Motorgehäuse (1) mit Ausnehmungen (28, 29, 30, 31) versehen sind, in die die Stößel (4, 9) wechselweise eingreifen, und der Hülse (17) in axialer Richtung ein Stößel (14) zum Betätigen der Zuführeinrichtung (3, 8) für das Druckmittel zugeordnet ist.

3. Nadelloses Injektionsgerät nach Anspruch 2, dadurch gekennzeichnet, daß der teleskopartig gefederte Stößel (4) Anschläge (46, 47, 48) zum Begrenzen seiner Auslenkung aufweist.

## Claims

1. A needle-less injection instrument with a piston pump (A) for the medium to be injected, which piston pump (A) is connected to a drive motor (B), of which the working piston (5), supported on a working spring (2), is arranged displaceably in a cylindrical bore (6) of the motor housing (1), there being formed in the cylindrical bore (6) a pressure chamber (7) for receiving a pressure medium to tension the working spring (2), which pressure chamber (7) has supply (3, 8) and discharge devices for the pressure medium, the working spring (2) being held in a tensioned position by a pawl (10) supported by a bolt (13), and the bolt (13) being supported on an operating element (C), wherein the operating element (C) is connected rigidly to the bolt (13), and the bolt (13) is arranged displaceably in the motor housing (1).

2. The needle-less injection instrument as claimed in claim 1, wherein the operating element (C) consists of a spring-loaded operating knob (16) which is engaged with a spring-loaded sleeve (17) arranged concentrically to the operating knob (16), a telescopically sprung plunger (4) being assigned to the sleeve (17) and a rigid plunger (9) being assigned to the operating knob (16), the two plungers (4, 9) being guided in appropriate bores (42, 43) in the operating element (C) and being supported by springs (32, 33, 34), and the sleeve (17), operating knob (16) and motor housing (1) being provided with recesses (28, 29, 30, 31) into which the plungers (4, 9) engage alternately, and a plunger (14) for actuating the supply device (3, 8) for the pressure medium being assigned to the sleeve (17) in an axial direction.

3. The needle-less injection instrument as claimed in claim 2, wherein the telescopically sprung plunger (4) has stops (46, 47, 48) for limiting its deflection.

## Revendications

1. Appareil d'injection sans aiguille comprenant une pompe à piston (A) pour le milieu à injecter, la pompe à piston (A) étant reliée à un moteur d'entraînement (B) dont le piston travaillant (5), qui prend appui sur un ressort travaillant (2) est monté coulissant dans un alésage cylindrique (6) du corps (1) du moteur, une chambre de pression (7) destinée à recevoir un fluide sous pression pour l'armement du ressort travaillant (2) étant formée dans l'alésage cylindrique (6), la chambre de pression (7) présentant des dispositifs d'arrivée (3, 8) et d'évacuation pour le fluide sous pression, le ressort travaillant (2) étant retenu dans la position armée par un cliquet (10) qui est soutenu par un verrou (13), et le verrou (13) s'appuyant sur un élément de manoeuvre (C), cet appareil étant caractérisé en ce que l'élément de manoeuvre (C) est relié rigidement au verrou (13) et que le verrou (13) est monté mobile en translation dans le corps (1) du moteur.

2. Appareil d'injection sans aiguille selon la revendication 1, caractérisé en ce que l'élément de manoeuvre (C) est constitué par un bouton de manoeuvre (16) chargé par un ressort, qui se trouve en prise avec une douille (17) chargée par un ressort et disposée concentriquement au bouton de manoeuvre (16), un poussoir télescopique à ressort (4) étant associé à le douille (17) et un poussoir rigide (9) étant associé au bouton de manoeuvre (16), les deux poussoirs (4, 9) étant guidés dans des alésages correspondants (42, 43) formés dans l'élément de manoeuvre (C) et étant soutenus par des ressorts (32, 33, 34), la douille (17), le bouton de manoeuvre (16) et le corps (1) du moteur étant munis d'évidements (28, 29, 30, 31) dans lesquels les poussoirs (4, 9) s'engagent alternativement et un poussoir (14) destiné à l'actionnement du dispositif d'arrivée (3, 8) du fluide sous pression étant associé à la douille (17) dans la direction axiale.

3. Appareil d'injection sans aiguille selon la revendication 2, caractérisé en ce que le poussoir télescopique à ressort (4) présente des butées (46, 47, 48) servant à limiter son excursion.

FIG. 2

FIG. 1

0 063 340

FIG. 4

FIG. 3

FIG. 5